# EUROPEAN PATENT SPECIFICATION

(11) **EP 2 803 274 B1**
(45) Date of publication and mention of the grant of the patent: **25.05.2016**
(21) Application number: 13735749.7
(22) Date of filing: 11.01.2013
(51) Int. Cl.: A23D 9/02, A23D 9/007

(54) **METHOD FOR PRODUCING LACTONE-ENRICHED OIL OR FAT COMPOSITION**
VERFAHREN ZUR HERSTELLUNG EINER MIT LACTON ANGEREICHERTEN ÖL- ODER FETTZUSAMMENSETZUNG
PROCÉDÉ DE PRODUCTION D'UNE COMPOSITION D'HUILE OU DE GRAISSE ENRICHIE EN LACTONE

(30) Priority: 11.01.2012 JP 2012003253
(43) Date of publication of application: 19.11.2014
(73) Proprietor: Kyushu University, National University Corporation, Fukuoka-shi, Fukuoka 812-8581 (JP)
(72) Inventor: SHIMODA, Mitsuya, Fukuoka-shi Fukuoka 812-8581 (JP)
(74) Representative: Hoffmann Eitle
(86) International application number: PCT/JP2013/050362
(87) International publication number: WO 2013/105624

(56) References cited:
- EP-A1- 1 538 215
- WO-A1-2008/032852
- JP-A- H0 994 062
- JP-A- S58 146 252
- JP-A- S58 146 252
- JP-A- 2009 261 339
- JP-A- 2009 261 339
- JP-A- 2010 189 513
- US-A1- 2009 258 128
- SABINE WIDDER ET AL: "Changes in the flavour of butter oil during storage", ZEITSCHRIFT F&#XFFFD;R LEBENSMITTEL-UNTERSUCHUNG UND -FORSCHUNG, vol. 193, no. 1, 1 January 1991 (1991-01-01), pages 32-35, XP055159407, ISSN: 0044-3026, DOI: 10.1007/BF01192013
- ALEWIJN ET AL: "The formation mechanism of lactones in Gouda cheese", INTERNATIONAL DAIRY JOURNAL, ELSEVIER APPLIED SCIENCE, BARKING, GB, vol. 17, no. 1, 9 November 2006 (2006-11-09), pages 59-66, XP005755153, ISSN: 0958-6946, DOI: 10.1016/J.IDAIRYJ.2006.01.002
- WIDDER S. ET AL.: 'Changes in the flavour of butter oil during storage.' Z LEBENSM UNTERS FORSCH vol. 193, pages 32 - 35, XP055159407
- MALLIA S. ET AL.: 'Aroma-active compounds of butters: a review.' EUR FOOD RES TECHNOL vol. 226, 2008, pages 315 - 325, XP019584946
- TOMOMI NAKAMURA ET AL.: 'Butter Yushi no Flavor Zokyo ni Kansuru Kenkyu' THE 59TH PROCEEDINGS OF JAPANESE SOCIETY OF FOOD SCIENCE AND TECHNOLOGY vol. 59TH, 29 August 2012, page 198, XP008174430

## Description

### TECHNICAL FIELD

The present invention relates to a method for improving flavor of an oil and fat composition, especially, butter. The present invention is useful in the fields of the oil and fat processing, the food production and so on.

### BACKGROUND ART

It was known in the art that, for enhancing butter flavor and milk flavor, milk fat is hydrolized by lipolytic enzymes such as lipase or esterase (Patent Document 1, and Non-Patent Document 1). Further, for the purpose of generating butter flavor, with suppressed oxidization odor or irritating odor which had been accompanied by the conventional butter flavor, with increased contents of lactones or ketones, and with favorable butter flavor, a method for preparing butter flavor by 2-step enzymatic decomposition was developed in the art wherein the method comprises the first hydrolysis step using at least one lipolytic enzymes and then the second hydrolysis step using at least one lipolytic enzymes (Patent Document 2).

On the other hand, on the basis of the fact that a balanced butter flavor can be conferred to food products by supplemented with oxidized butter fat, a method for producing a food product with flavor being conferred had been developed in the art, wherein the method is characterized by adding to edible materials at least 0.05% (weight/weight) of oxidized butter fat containing 0.5 ppm or more of n-pentanal content and/or 0.05 ppm or more of 2-trans-nonenal content (Patent Document 3). Further, focusing on the change of flavor caused by oxidization of butter fat, for the purpose of improving butter flavor caused by the previous enzymatic decomposition, a method for producing butter flavor with enhanced milk flavor and enhanced butter flavor, wherein the method is characterized by a step of hydrolysis of milk fat using lipolytic enzymes and then a step of oxidization through ultraviolet irradiation (Patent Document 4). Further, it was reported that δ-decalactone concentration level in butter was increased from 1193 to 2633 ppb, by heat-treating of melted butter at 105 - 110°C for 15 minutes, which resulted in improvement of flavor (Non-Patent Document 2 and 3).

It is considered to be preferable that more contents of lactones or ketones are contained as butter flavor (forecited Patent Document 2). Specifically, it was reported in the art that non-fermented butter (sweet cream butter) is characterized by lactones associated with fruity and creamy note, on the other hand, key compounds of fermented butter are diacetyl (butter like), butanoic acid (cheese like), and δ-decalactone (peach like) generated by mainly lactobacillus fermentation, and butter oil is characterized by aldehydes such as (E)- or (Z)-2-nonenal and (E,E)-2,4-decadienal which are also referred to as green note and oily note (Non-Patent Document 4).

### CITAITON LIST

### PATENT DOCUMENTS

[Patent Document 1] Japanese Patent Public Disclosure No. S64-2549 (Japanese Patent Examined Patent Publication No. H07-083686)
[Patent Document 2] Japanese Patent Public Disclosure No. 2009-261339
[Patent Document 3] Japanese Patent Public Disclosure No. S64-39962
[Patent Document 4] Japanese Patent Public Disclosure No. H09-094062

### NON-PATENT DOCUMENTS

[Non-Patent Document 1] Balcao VM, and Malcata FX, Lipase catalyzed modification of milkfat. Biotechnology Advances, Vol. 16, No. 2, pp. 309-341, 1998.
[Non-Patent Document 2] Peterson DG, and Reineccius GA, Determination of the aroma impact compounds in heated sweet cream butter. Flavour and Fragrance Journal, Vol. 18, pp. 320-324, 2003.
[Non-Patent Document 3] Peterson DG, and Reineccius GA, Chracterization of the volatile compounds that constitute fresh sweet cream butter aroma. Flavour and Fragrance Journal, Vol. 18, pp. 215-220, 2003.
[Non-Patent Document 4] Mallia S, et al., Aroma-active compounds of butter. European Food Research and Technology, Vol. 226, pp. 315-325,2008.

### SUMMARY OF INVENTION

### TECHNICAL PROBLEM

It was known that 97 - 98% of butter-composing lipid, are triglycerides that are prepared by ester linkage between various kinds of fatty acids and glycerol. Lactones contained in milk fat are key compounds of the butter flavor, while the precursor materials of lactones are 4- or 5-hydroxy fatty acids, which are linked with glycerol via ester linkage. When lipolytic enzymes such as lipase or esterase are used to hydrolyze hydroxy fatty acid ester to generate lactones, overwhelmingly higher amount of lower fatty acids, intermediate fatty acids and higher fatty acids are also generated at the same time. Further, irritating odor derived from volatile lower fatty acids, and wax odor derived from the intermediate fatty acids and the higher fatty acids become problems. Moreover, it is necessary to finally deactivate the enzymes used, where the deactivation is generally achieved by heat treatment such as heat treatment at 80°C for 20 minutes. During the heat treatment, butter flavor has become deteriorated.

### SOLUTION TO PROBLEM

The present invention provides a method for non-enzymatically enriching lactones in the oil and fat composition. The present inventors thought that ester degradation reaction of the hydroxy fatty acid which forms ester linkage with glycerol and lactone-forming reaction were proceeded through catalytic activity of the water molecules under mild condition. That is to say, the present inventors thought that there is a reaction mechanism where linkage formation between oxygen in hydroxyl group and acyl carbon, proton elimination from the hydroxyl group, proton-donating for oxygen associated with ester linkage, and cleavage of ester bonding proceed at the same time. On the other hand, the present inventors also thought that under the presence of excess water molecules, linkage formation between the hydroxyl group and the acyl carbon is inhibited, which results in slowing lactone generation speed. As a result, the inventors reached a conclusion that, when a catalytic amount of moisture exists in the oil and fat composition containing 4- or 5-hydroxy fatty acid ester, it is possible to selectively produce lactones by keeping the oil and fat composition under mild condition.

Further, the present inventors completed one embodiment of the present invention on the basis of the teaching that it is possible to obtain butter oil with enhanced lactone content and with superior flavor by adjusting the moisture content in the butter oil and placing the butter oil at temperature of 20°C (solid fat content is about 26%) for a few weeks.

The present inventors also completed another embodiment of the present invention on the basis of the teaching that it is possible to obtain butter oil with enhanced lactone content and with superior flavor by adding sucrose to the oil and fat composition and then keeping the butter oil at temperature of 40 - 60°C for 2 - 4 hours.

The present invention provides the following embodiments:
[1] A method for producing an oil and fat composition enriched with lactones, which comprises steps of:
   keeping the oil and fat composition containing between 300 ppm and 1200 ppm of moisture content at the temperature where a solid fat exists in the oil and fat composition, and
   non-enzymatically producing γ-lactone or δ-lactone from triglyceride containing 4- or 5-hydroxy fatty acid in the oil and fat composition.
[2] The method for producing the oil and fat composition according to [1], further comprising a step of placing the raw material oil and fat composition in the melted state under reduced pressure and/or a step of centrifuging the raw material oil and fat composition in the melted state to remove moisture to obtain the oil and fat composition containing between 300 ppm and 1200 ppm of moisture content.
[3] The method for producing the oil and fat composition according to [1], further comprising a step of adding moisture to the raw material oil and fat composition to obtain the oil and fat composition containing between 300 ppm and 1200 ppm of moisture content.
[4] The method for producing the oil and fat composition according to any one of [1]-[3], wherein the oil and fat composition is derived from butter.
[5] The method for producing the oil and fat composition according to any one of [1]-[4], wherein the temperature is kept between 10°C and 35°C, or more preferably between 15°C and 30°C.
[6] The method for producing the oil and fat composition according to any one of [1]-[5], wherein the oil and fat composition is held between 3 days and 2 months, or more preferably between 1 week and 4 weeks.
[7] The method for producing the oil and fat composition according to any one of [1]-[6], which is for the purpose of producing the oil and fat composition containing 50 ppm or more of lactones.
[8] A method for producing a food product or a food product material, which comprises steps described in any one of [1]-[7], wherein the oil and fat composition is enriched with lactones.
[9] A method for producing an oil and fat composition enriched with lactones, which comprises steps of:
   adding to the oil and fat composition an additive agent which has an ability to form hydrogen bonds with water molecules,
   keeping temperature of the oil and fat composition at the temperature between 40 and 60°C,
   non-enzymatically producing γ-lactone or δ-lactone from triglyceride containing 4- or 5-hydroxy fatty acid in the oil and fat composition.
[10] The method for producing the oil and fat composition according to [9], wherein the additive agent is sugar selected from at least one selected from the group consisting of monosaccharides, disaccharides, oligosaccharides, dextrins, and starches.
[11] The method for producing the oil and fat composition according to [10], wherein the sugar is sucrose.
[12] The method for producing the oil and fat composition according to any one of [9]-[11], wherein the oil and fat composition is derived from butter.
[13] The method for producing the oil and fat composition according to any one of [9]-[12], wherein the oil and fat composition is held between 2 to 4 hours, or more preferably for 3 hours.
[14] The method for producing the oil and fat composition according to any one of [9]-[13], which is for the purpose of producing the oil and fat composition containing 50 ppm or more of lactones.

### ADVANTAGEOUS EFFECTS OF INVENTION

According to the present method, it is possible to produce flavor of good quality without necessity of using expensive enzymes.

In the method of the present invention, it may not be necessary to use a lipolytic enzyme. As a result, it is possible to improve the desired flavor, while suppressing generation of irritating odor or wax odor causing quality loss of flavor. Further, since the method of the present invention is non-enzymatically carried out under the mild condition, it is possible to suppress lactone polymerization reaction caused by excessive ester exchange reaction, resulting in accumulating lactones in the products.

According to the present invention, it is possible to convert almost all precursors of lactones included in butter into the lactones, when using the butter as the raw material oil and fat composition.

### BRIEF DESCRIPTION OF DRAWINGS

Fig. 1 is a diagram showing production of δ-lactone from 5-hydroxy fatty acid ester.
Fig. 2 is a graph showing lactone concentrations when Sample B was stored at 20°C.
Fig. 3 is a graph showing lactone concentrations when Sample D was stored at 20°C.
Fig. 4 is a graph showing lactone concentrations when Sample E was stored at 20°C.
Fig. 5 is a graph showing effects of moisture contents on lactone concentrations of Samples which were stored at 20°C.
Fig. 6 is a graph showing productions of fatty acids during storing Sample B at 20°C.
Fig. 7 is a graph showing productions of fatty acids during storing Sample C at 20°C.
Fig. 8 is a graph showing lactone concentrations when Sample D was stored at 5°C.
Fig. 9 is a graph showing effects of holding temperatures on total lactone concentration of Samples.
Fig. 10 is a graph showing transition of lactone concentrations during keeping the temperature of Sample G at 50 or 60°C.

### DESCRIPTION OF EMBODIMENTS

The present invention provides a method of non-enzymatically producing an oil and fat composition enriched with lactones.

In the present invention, the term "oil and fat composition" indicates, unless otherwise specifically described, a composition containing as a main component, an ester of a fatty acid and a glycerin (in other words, triglyceride, triacylglycerol, tri-O-acylglycerin). In the present invention, the term "main component" relating to the oil and fat composition means a component which accounts for 90% or more by weight. In the present invention, the term "fatty acid" means, unless otherwise specifically described, monocarboxylic acids containing 4 or more carbons. In the present invention, the term "triglyceride" means, unless otherwise specifically described, any of triglycerides where the composed fatty acid is any one of lower fatty acids containing 6 or lower carbons, intermediate fatty acids containing 7 -10 carbons, and higher fatty acids containing more than 10 carbons (each referred to as the short chain fatty acid, intermediate chain fatty acid, and long chain fatty acid).

The oil and fat composition used in the present invention is preferably derived from butter, cream, butter oil, or cheese.

In the present invention, the term "butter (product)" means, unless otherwise specifically described, an edible oil and fat composition (product) which is prepared by separating fat contained in an animal milk and then being curdled. In the present invention, relating to the term "animal milk", its typical example is bovine milk. The term "butter (product)" of the present invention includes not only "butter (product)" prepared from bovine milk as a raw material but also "butter (product)" prepared from animal milk other than bovine milk as a raw material.

The butter of the present invention may be prepared by any method of producing the butter; generally, by a step of separating cream from the raw material milk by centrifugation, followed by a step of churning and solidification, and optionally including a step of lactate fermentation. The butter of the present invention includes a fermented butter (fermented cream butter) and a non-fermented butter (sweet cream butter). In other definition, the butter includes a salted butter to which salt is added and a non-salt butter (sometimes referred to as unsalted butter). The typical example of the butter contains about 80% of components of milk fat and water-in-oil emulsion comprising about 17% of moisture. Further, the butter contains saturated fatty acid at an amount of about 50%.

In the present invention, the term "cream" means, unless otherwise specifically described, a component typically containing about 18.0% or more of milk fat which is prepared by eliminating components other than the milk fat from animal milk.

In the present invention, the term "butter oil" means, unless otherwise specifically described, a component which is prepared by eliminating almost all components other than milk fat from butter or cream.

In the present invention, the term "non-enzymatically" means, unless otherwise specifically described, that a reaction is conducted without using lipolytic enzymes such as lipase, elastase, and so on.

It was previously reported that by heat-treating the butter oil at 160°C for 1 - 2 hours in the presence of 5% moisture, lactones were quantitatively formed (Non-Patent Document: The formation mechanism of lactones in Gouda cheese. International Dairy Journal, Vol. 17, pp.59-66, 2007.).

Nevertheless, in the present invention, in the presence of an appropriate amount of the moisture, the ester degradation reaction and the lactone production reaction of the hydroxy fatty acid in the oil and fat automatically proceed under the mild condition without enzymatic activity. It has not yet been reported in the prior art that lactone production may proceed through the catalytic activity of water molecules under the mild condition, without the presence of enzymes such as lipase.

The present inventors conducted various conditions relating to lactone production using lipase and found that a large amount of the volatile fatty acid is liberated when the moisture content in the butter is high, and that, when a small amount of lipase is added to the dehydrated butter oil, lactone production with intense aroma is sensed within about 1-3 minutes after addition of an enzyme, and the lactone aroma is significantly reduced after 5-7 minutes after addition of an enzyme. It was considered that lactones which were produced in the dehydrated butter oil, were subjected to various ester exchange reaction by enzymes. The method using lipase may be considered to be an effective means if it is possible to completely deactivate lipase or completely separate at the time when the amount of lactone production is optimum, but however is considered to be practical. On the other hand, in the method of the present invention, reaction is non-enzymatical and lactone production is gently proceeded.

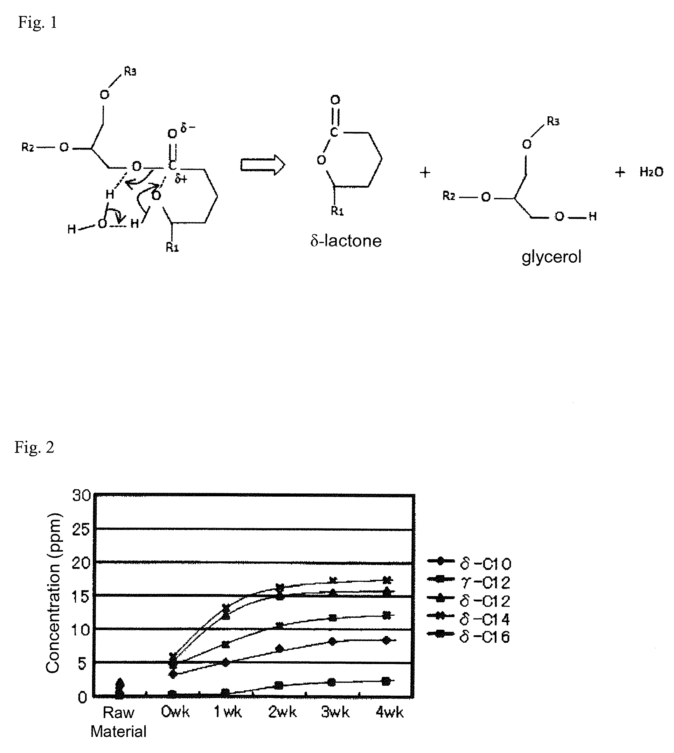

More specifically, in one embodiment of the present invention, linkage formation between acyl carbons which is bound via ester linkage to an oxygen of the hydroxyl group of the triglyceride-composing hydroxy fatty acid, proton elimination from the hydroxyl group, proton-donating for oxygen associated with ester linkage, and cleavage of ester bonding are proceeded at the same time under the mild condition through a step of forming a reaction intermediate mediated by one molecule of water. In this embodiment, the presence of excess water molecules inhibits formation of the reaction intermediate, which results in slowing the lactone production. A production reaction from 5-hydroxy fatty acid ester to δ-lactone is demonstrated in Fig. 1 as a typical example of this reaction.

A triglyceride contains three acyl carbons which are associated with ester linkage. Although it is quite unlikely to progress reactions on the positions 1, 2 and 3 at equivalent probability due to the steric hindrance, the lactone production reaction will possibly be occurred on any of positions 1, 2 and 3 as far as hydroxy fatty acid is associated with ester linkage.

On the other hand, the hydroxy group of the hydroxy fatty acid can be used for alcoholysis the ester bonding of other triglyceride molecule. Therefore, for optimizing lactone production reaction, it may be preferable to select the condition where lactone production reaction that is an alcoholysis reaction within a molecule is preferentially proceeded while alcoholysis between molecules is suppressed.

In one embodiment of the present invention, the lactone production reaction is proceeded in the presence of a small amount of moisture. Therefore, it is necessary to adjust moisture content in the raw material oil and fat composition (removal or addition of moisture). Under the condition where removal of moisture is insufficient and the moisture content in the raw material oil and fat composition is relatively high, it is considered that nucleophilicity of the hydroxyl group of the hydroxy fatty acid to the acyl carbon is relatively low. Further, according the study conducted by the present inventors, in the butter oil in which the moisture content is adjusted below a certain level, specifically at 280 ppm, the lactone production resulted in a significant reduction. This demonstrates that the presence of an appropriate amount of moisture is indispensable for the lactone production from the hydroxy fatty acid.

Those ordinarily skilled in the art could have carried out the removal of moisture from the oil and fat composition using similar procedures which are generally used in the art for similar purpose. For example, it is possible to remove a majority of moisture by melting the raw material oil and fat composition, centrifuging sufficiently, separating and removing the water phase. For further reducing the moisture content, the melted raw material oil and fat composition may be placed under reduced pressure. For reference, the term "melted" relating to the oil and fat composition of the present invention means that, unless otherwise specifically described, an amount of the melted oil and fat (the melted fat) contained in the oil and fat composition is 80% (weight ratio) or more. In the present invention, a melting condition which is preferable for the dehydration process is temperature where the amount of the melted fat is 90% or more, or more preferably, temperature where the amount of the melted fat is 99% or more. It is known that an amount of the solid fat in the butter oil is 26.4% at 20°C, 25% at 25°C, 21% at 30°C, 7.4% at 35°C, and 0% at 40°C (J. Am. Oil Chem. Soc. Vol. 87, pp. 493-497(2010)). Further, it is also known that an amount of the solid fat in the butter oil is 32% at 15°C, 44% at 10°C, 56% at 5°C (J. Am. Oil Chem. Soc. Vol. 70, pp. 1193-1201(1993)). Therefore, when dehydration of the butter oil is carried out in the present invention, it is preferable to carried out the process at 30°C or higher, more preferably at 35°C or higher, further preferably at 38°C or higher.

Those ordinarily skilled in the art could also have carried out the addition of moisture to the oil and fat composition using various similar procedures which are generally used in the art for similar purpose. Sometimes, the moisture content of the oil and fat composition may be adjusted to be very low for securing preservative quality and stability. When such raw material oil and fat composition with very low moisture content is used in the present invention, moisture may be appropriately added to the oil and fat composition such that the moisture content may be appropriately adjusted to the given moisture content.

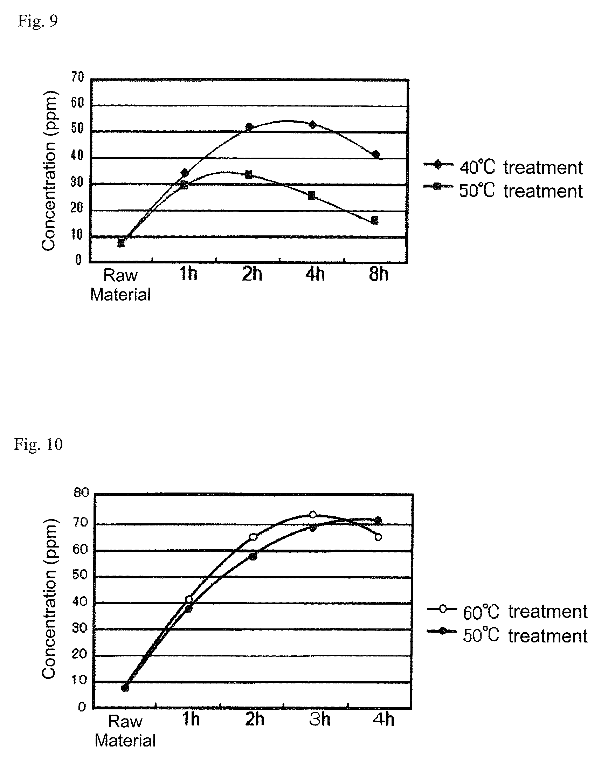

On the other hand, when the butter oil with adjusted moisture was hold at 40°C or 50°C, an amount of the lactone production reached maximum amount after 2 hours and then shifted to decline afterward (Fig. 10). It is considered that hydroxy fatty acid which consists of triglyceride was consumed by the reaction other than lactone production reaction in the raw material oil and fat composition. That is to say, since, when the raw material oil and fat composition is apparently solid, lactone is preferentially produced through the intramolecular reaction, while in the melting condition that is an environment where triglyceride molecules can relatively freely moved, it is considered that the probability of the intermolecular reaction is increased.

From this viewpoint, it is desirable that the process of eliminating the moisture in the melting condition is carried out within a short time. According to the study in the present inventors, when a process using rotary evaporator under the reduced pressure (at 40°C), or an equivalent process can be used to eliminate the moisture from the raw material oil and fat composition to 280 ppm within about 20 minutes. Generally, the condition where the moisture is eliminated from the raw material oil and fat composition in the present invention, under the reduced pressure, is preferably at 35°C or higher, is more preferably at 38°C or higher. In each condition, the temperature of 45°C or lower is preferable, the temperature of 42°C or lower is more preferable, and the temperature of 40°C or lower is further preferable. Please note that, in the present invention, the temperature used in the process means temperature of the environment where the oil and fat composition is placed, which is generally the same temperature as the oil and fat composition itself.

In one embodiment of the present invention, the moisture content contained in the raw material oil and fat composition is required to be set at 300 ppm or higher, for example, preferably at 400 ppm, further preferably at 520 ppm or higher. Further, in each case, the moisture content is 1200 ppm or less, or may be preferably at 1000 ppm or less. The moisture content in the oil and fat composition can be measured using the conventional method for measuring the moisture content in food and drink products, such as Karl Fischer's method.

In one embodiment of the present invention, lactones are automatically generated by placing the raw material oil and fat composition, the moisture content of which is adjusted to between 300 ppm and 1200 ppm, under the temperature effective for lactone production for the period effective for lactone production.

In one embodiment, more specifically, the temperature effective for lactone production is the temperature where the raw material oil and fat composition seems to apparently be solid state or where the solid fat content seems to be relatively high, and where the sufficient amount of solid fat exists in the oil and fat composition. In the melting condition, the triglyceride molecules can move relatively freely and can easily generate intermolecular reaction (alcoholysis); while the ratio of lactone production which is achieved by intramolecular reaction increase in the solid state, since the diffusion of triglyceride molecules is suppressed in the solid state. Therefore, in the present invention, the upper limit of the temperature effective for lactone production corresponds to the temperature where there are solid fat in the oil and fat composition, more preferably, the temperature where 7% or more of the solid fat is contained, further preferably, the temperature where 20% or more of the solid fat is contained. In one embodiment of the present invention, in the case where the butter oil is used as the raw material oil and fat composition part, on the basis of the ratio of the solid fat in butter in each temperature described above, the processing temperature is preferably 35°C or lower, or more preferably 30°C or lower.

From this viewpoint, the condition of lactone production step in the first embodiment of the present invention is more preferably 10°C or higher and, is more preferably 15°C or higher. In each case, the temperature is preferably 35°C or lower, or is more preferably 30°C or lower.

In one embodiment, the period effective for lactone production may be 3 days or longer, more preferably, 1 week or longer, further preferably, 2 weeks or longer. As described in Table 1, the lactones, the amount of which are comparable to the lactone potential of the oil and fat (i.e., the maximum amount of the lactone production, International Dairy Journal, Vol. 17, pp. 59-66(227)) was accumulated within three weeks. The oxidization reaction of the oil and fat tends to proceed as the period would be longer. Therefore, the period will be two months or less, preferably 4 weeks or less, and more preferably 3 weeks or less.

In one embodiment of the present invention, by placing the raw material oil and fat composition containing a small amount of moisture under the relatively mild condition, the ester degradation reaction of the hydroxy fatty acid which consists of triglyceride and lactone production reaction can be non-enzymatically proceeded. On the other hand, it is difficult to undergo hydrolysis of the ester bonding of the fatty acid without a hydroxy group at 4 position or 5 position under the conditions of the present invention. That is to say, according to the present invention, it is possible to suppress production of unfavorable free fatty acid and selectively produces lactones.

Further, the present invention conducted further study for the purpose of soving the time-consuming issue which is a problem of the first embodiment of the present invention. As a result, the inventors found that it is possible to produce a substantial amount of lactones in the oil and fat composition, which was relevant to the amount obtained by the first embodiment and which was achieved within a remarkably short period of time as compared with the first embodiment, without strictly controlling the moisture content, by mixing, in the oil and fat composition, an additive agent having an ability to form a hydrogen bond with a water molecule, and appropriately adjusting the temperature conditions.

It is possible to apply as the above described additive agents, organic compounds (such as monosaccharides, disaccharides, oligosaccharides, dextrins, starches), inorganic compounds (such as silica gel), and any combination thereof.

Specifically, in the second embodiment of the present invention, it is possible to produce the lactones-enriched oil and fat composition through production of non-enzymatically γ-lactone, or δ-lactone from triglycerides including 4- or 5-hydroxy fatty acids in the oil and fat composition, by adding sucrose in the oil and fat composition and keeping the temperature between 40 - 60°C.

While not wishing to be bound by any particular theory, the inventors discovered that enrichment of lactones in the second embodiment was achieved by binding the sucrose molecules contained in the oil and fat composition to the water molecules in the oil and fat composition, which moderately suppresses the hydrolysis ability of the water molecules.

The second embodiment does not require to conduct a step of adjusting moisture associated with a treatment under the reduced pressure, that is required in the first embodiment, and, as a result, as compared with the first embodiment, exhibits a significant effect of possibility that the lactones in the oil and fat composition can be enriched within a remarkably short period of time. Further, since the reaction time can be shorten, it is possible to further reduce emergence of irritating odor or wax odor that causes quality loss of flavor.

In the second embodiment, more specifically, the temperature effective for lactone production ranges between 40°C - 60°C, and more preferably, is 50°C. In this embodiment, the reaction may be continued under the temperature condition as described above for about 2 - 4 hours, more preferably for about 3 hours, for achieving the lactone production.

In the conventional method using a lipolytic enzyme, in addition to hydroxy fatty acids necessary for lactone production, a large amount of by-products (such as lower fatty acids, intermediate fatty acids and higher fatty acids) are concurrently generated, which elicits problematic emergence of irritating odor which is derived from volatile lower fatty acids or wax odor which is derived from intermediate fatty acids and higher fatty acids. Further, it is necessary to conduct heat treating for deactivating the enzymes used, which causes a problem of deterioration of flavor.

The lactones-enriched oil and fat composition obtained by the present invention suppresses irritating degradation odor or oxidization odor and enhances favorable butter flavor, as compared with the conventional butter flavor. The lactones-enriched oil and fat composition obtained by the present invention may contain 50 ppm or more of lactones. More preferably, the lactones-enriched oil and fat composition obtained by the present invention may contain 60 ppm or more of lactones. It is preferable that the lactones-enriched oil and fat composition contains, as lactones, 1, 2, or more of materials such as δ-decalactone, γ-dodecalactone, δ-dodecalactone, δ-tetradecalactone, and δ-hexadecalactone, and it is especially preferable that the lactones-enriched oil and fat composition may be high in the content of δ-decalactone δ-dodecalactone and δ-tetradecalactone.

The lactones-enriched oil and fat composition obtained by the first embodiment of the present invention may have the characteristics selected from (1) - (4) and any combinations of (1) - (4) as listed below:
(1) Containing lactones at 50 ppm, or preferably 60 ppm or more.
(2) Containing lactones, at an amount of which are comparable to the lactone potential of the raw material oil and fat composition, for example, 90% or more of the lactone potential of the raw material oil and fat composition, preferably, 95% or more of the lactone potential of the raw material oil and fat composition. In other words, the composition contains little amount of 4- or 5-hydroxy fatty acid which is linked to glycerol through the ester linkage.
(3) Containing the moisture at 280 ppm or more; for example, preferably at 300 ppm or higher; more preferably at 400 ppm of the moisture content; further preferably 520 ppm or higher of the moisture content. Further, in each cases, the moisture content may be preferably at 10000 ppm or less, may be preferably at 1300 ppm or less, may be more preferably at 1200 ppm or less, or may be further preferably at 1000 ppm or less.
(4) Containing a reduced amount of unfavorable fatty acid, more specifically, irritating odor which is derived from volatile lower fatty acids and wax odor which is derived from intermediate fatty acids and higher fatty acids. Especially, it is preferable that an amount of each fatty acid, C10, C12, or C 14, in the lactones-enriched oil and fat composition is almost the same as an amount of each fatty acid in the raw material oil and fat composition, or that, specifically, when the fatty acid content (weight) of the raw material oil and fat composition is considered to be 100, the amount of each fatty acid in the lactones-enriched material is 120% or less, preferably 110% or less, further preferably 105% or less.

The lactones-enriched oil and fat composition obtained by the second embodiment of the present invention may have the characteristics selected from (1) - (4) and any combinations of (1) - (4) as listed below:
(1) Containing lactones at 50 ppm, or preferably at 60 ppm or more.
(2) Containinng lactones, at an amount of which are comparable to the lactone potential of the raw material oil and fat composition, for example, 90% or more of the lactone potential of the raw material oil and fat composition, preferably, 95% or more of the lactone potential of the raw material oil and fat composition. In other words, the composition contains little amount of 4- or 5-hydroxy fatty acid which is linked to glycerol through the ester linkage.
(3) Containing sucrose at 0.5% - 6.0%, more preferably at 1.0 - 6.0%, further preferably at 2.0 - 6.0% in the composition.
(4) Containing a reduced amount of unfavorable fatty acid, more specifically, irritating odor which is derived from volatile lower fatty acids and wax odor which is derived from intermediate fatty acids and higher fatty acids. Especially, it is preferable that an amount of each fatty acid, C10, C12, or C14, in the lactones-enriched oil and fat composition is almost the same as an amount of each fatty acid in the raw material oil and fat composition, or that, specifically, when the fatty acid content (weight) of the raw material oil and fat composition is considered to be 100, the amount of each fatty acid in the lactones-enriched material is 120% or less , preferably 110% or less, further preferably 105% or less.

Further, the present invention is also superior in terms of the fact that the lactones-enriched oil and fat composition under the mild condition without enzymes.

The food product or food product material using the oil and fat composition obtainable by the present invention maintains the characteristics of the oil and fat composition. That is to say, the food product or food product material using the lactones-enriched oil and fat composition obtainable by the present invention also has the characteristics selected from (1) - (4) described above or any combinations of (1) - (4) as listed above.

Also, "the lactones-enriched oil and fat composition obtainable by the preparation method" in the present invention, unless otherwise specifically described, includes not only the oil and fat composition obtained by the preparing method, but also any oil and fat composition obtained by other method.

The lactone generated by the method of the present invention is susceptible to hydrolysis at acid pH region and alkali pH region, however is relatively stable against heating or moisture. Therefore, the lactones-enriched oil and fat composition obtained by the present invention may be sufficiently stable during the usual food products processing. Therefore, the oil and fat composition obtained by the present invention may be used as it is as food products, or may be used as food products material added to various food products.

The "food products" or "food products material" nay be in the form of cake, as well as in the form of liquid such as a drink or soup. The term "food products material" includes various additives which may be acceptable as food products.

The present invention may be applied for enhancing flavor of milk products, especially cream or butter. Further, other milk products, such as butter oil, cheese , condensed whey, ice creams (ice cream, ice milk, ice cream with milk-solids content of 3% or greater, condenced milk (defatted concentrated milk, unsweetened condensed milk, unsweetened defatted condensed milk , sweetened condensed milk, sweetened defatted condensed milk), entire powdered milk, powdered skim milk, cream powder, whey powder, protein condensed whey powder, butter milk powder, sweetened powdered milk, conditioned powdered milk (such as powdered milk for infant), fermented milk, lactic acid bacteria beverage, milk beverage (coffee milk, enriched milk with calcium), sour cream (fresh cream which is subjected to lactate fermentation). The present invention can also be applied to the food products which are desirable to enhance flavor of cream or butter. These food products include, but not limited to, bakery products, confectionery products, chocolate products, egg products such as omelette, processed oil and fat products (margarine or hydrogenated fat, shortening), mayonnaise, or dressing.

The lactones-enriched oil and fat compositionor lactone obtained by the present invention can be used as an aroma material for being added to the food products.

Generally, since a compound which is prepared by the usual synthetic approach and is concerned about residues such as the starting material, the catalytic agent, or by-products, it is considered in the art to be preferable that an amount of the synthetic compound added to the food products is as small as possible. On the other hand, the lactone concentration in the natural products is very low and the lactone is also an important compound in the field of the aroma materials. Since the present invention can produce lactones by the spontaneous reaction without use of an enzyme, the present invention is also useful from this viewpoint.

### EXAMPLES

### [Example 1] Relations between moisture content and lactones in butter

Unsalted butter was commercially purchased and used. Unsalted butter as a raw material 200 g was melted in a thermostated chamber at a temperature of 40°C, placed it for 30 minutes or longer, then separated the upper layer to yield butter oil (Sample A). Sample A was centrifuged (3000 rpm, 3 minutes) to remove fine aqueous droplets and to yield Sample B. Sample A and Sample B contain 18600 ppm and 1222 ppm, respectively, which were determined by Karl Fischer's method using AQUACOUNTER, AQ-300 Karl Fischer Coulometric Titrator provided by HIRANUMA SANGYO Co., Ltd.. Further, rotary evaporator using reduced pressure (40°C) was used to conduct dehydration process for between a few minutes and 15 minutes, which resulted in adjusting the moisture content in the butter oil to 948 ppm (Sample C), 757 ppm (Sample D), 510 ppm (Sample E), 280 ppm (Sample F), respectively. Samples A, B, C, D, E and F were stored under a constant temperature of 20°C, for 0, 1, 2, 3, 4 weeks, concentrations of lactones and fatty acids contained in the butter oil of which were continuously measured.

For carrying out GC-MS analysis of the volatile components in the butter oil, it is necessary to selectively extract only lipophilic volatile materials from a large amount of nonvolatile or poorly-volatile oil and fat. In the prior art, Samples which were to be subjected to GC-MS analysis were only prepared only by the molecular distillation method (50°C, 0.1 mbar). In the present study, the present invention conceived of a simple method of producing Samples. That is to say, a pouch with a size of 6 centimeters by 5 centimeters was formed by heat-sealing of low-density polyethylene (LDPE) film, which was dipped in diethyl ether for 2 h or longer to eliminate the ether eluted material. Sample 20 g was yielded in the clean-upped pouch and the open end of the pouch was heat-sealed. The sealed pouch was transferred to 100 ml pressure-resistant sealed wide-mouth bottle, which was filled with 30 ml of diethyl ether and sealed, and then was subject to extraction on shaking at 40°C in a thermostated chamber for 1 hour. After completion of extraction and taking the pouch out, 5 mg of 4-n-butylchlorobenzene is added to the ether solution as an internal standard, which was concentrated to the given amount to prepare Samples for GC-MS (a product of Shimadzu Corporation, GCMS-QP2010 plus) analysis. The above described pretreatment process is called as LDPE pouch extraction process.

In order to determine lactones extraction efficiency (yield rate) by LDPE pouch extraction process, Samples which were prepared by supplementing a certain amount of δ-decalactone, γ-dodecalactone, δ-dodecalactone, δ-tetradecalactone, δ-hexadecalactone (products of WAKO CHEMICAL, LTD. or AAA ChemicalsInc.) to the butter oil, were subject to LDPE pouch extraction process and GC-MS analysis. Results showed that the every values of the lactone extraction efficiency for these samples ranges between 10 - 11 %. Then, the data were summarized on the basis of the fact that lactones yield rate from the Samples prepared by the LDPE pouch extraction process was deemed to be 10%.

Changes in concentrations of δ-decalactone (δ-C10), γ-dodecalactone (γ-C12), δ-dodecalactone (δ-C12), δ-tetradecalactone (δ-C14), δ-hexadecalactone (δ-C16) are exhibited in Fig. 2 when Sample B (containing 1220 ppm of moisture content) was stored at constant temperature of 20°C for 0, 1, 2, 3, or 4 weeks. Since it was difficult to determine the lactone concentration in the raw material butter oil by the LDPE pouch extraction process, values described in the Non-Patent Document (Characterization of the volatile compounds that constitute fresh cream butter aroma. Flavour and Fragrance Journal, vol. 18, pp. 215-220, 2003) were plotted (as well as Fig. 2 and other figures).

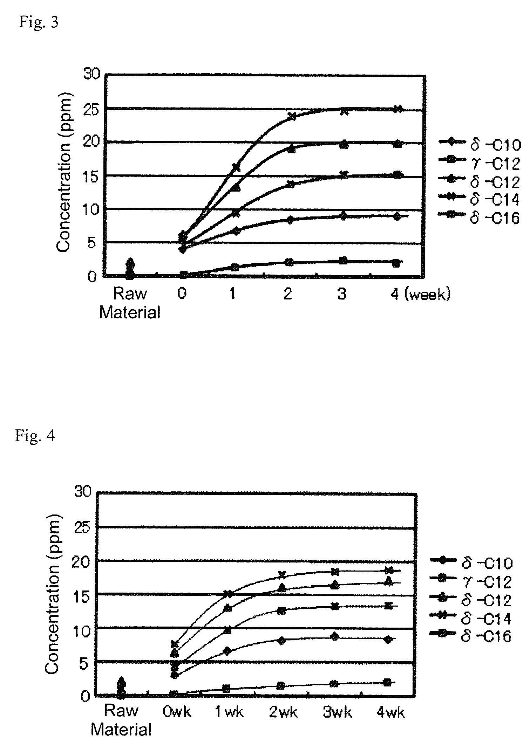

Since Sample C and Sample D (containing 948 ppm and 757 ppm of moisture contents, respectively) exhibit almost the same behavior in the lactone production, time-course change of Sample D is shown in Fig. 3. Compared with Fig. 2, increase in the concentration of lactones is significant. Fig. 4 shows time-course changes in concentration of Sample E (containing 510 ppm of moisture content), which shows that the production of lactones was relatively slow. From the results described above, it was confirmed that the moisture content in the butter oil has an effect on the production of lactones.

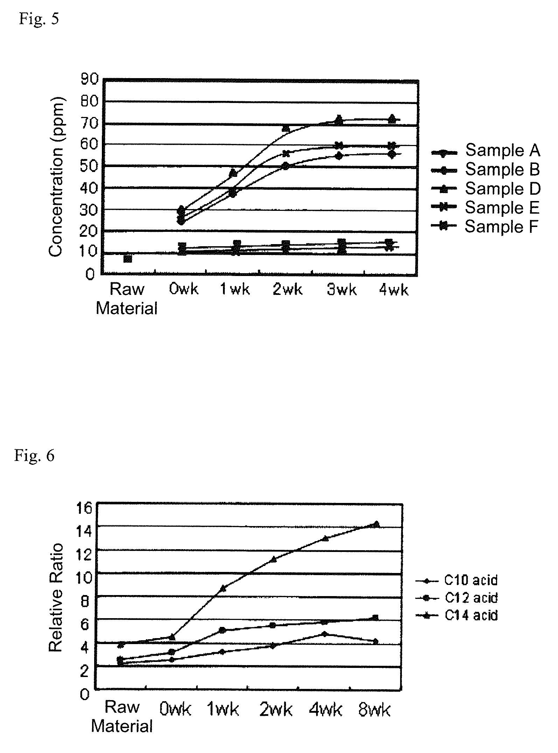

Fig. 2 (Sample B), Fig. 3 (Sample D) and Fig. 4 (Sample E) show changes in concentration of each lactone. Then, changes of total lactone contents were examined for Samples A-F, and Fig. 5 shows the changes of total lactone contents in Samples A, B, D, E, and F. From the data shown in Fig. 5, it was found that the highest accumulation of lactones was achieved in Sample D (containing 757 ppm of moisture content), where the level of lactones reached 70 ppm or more. It was found that Sample B (containing 1200 ppm or more of moisture) and Sample E (containing 510 ppm of moisture content) had a tendency to reduce the lactone production. Although not shown in the figures, it was shown that accumulated lactones in Sample C (containing 948 ppm of moisture content) was 44 ppm by measuring the total lactone content in Sample C by the similar procedure. Further, on the other hand, an amount of the accumulated lactones in Sample F (containing 280 ppm of the moisture content) and Sample A (containing 18600 ppm of moisture content) was very low.

It was considered that the reason why the accumulated lactones was low in Sample A was difficulties in production of the reaction intermediate shown in Fig. 1 under the high moisture content. It was considered that the fact that the lactone was seldom produced in Sample F demonstrates the absence of the water molecules which catalize lactone production reaction.

Thus, since it was found to be able to substantially stop the lactone production reaction by adjusting the moisture content in the butter oil to 280 ppm or less, the lactone extraction from Samples B, C, and D was conducted after dehydration of the moisture content to 280 ppm or less by the rotary evaporator.

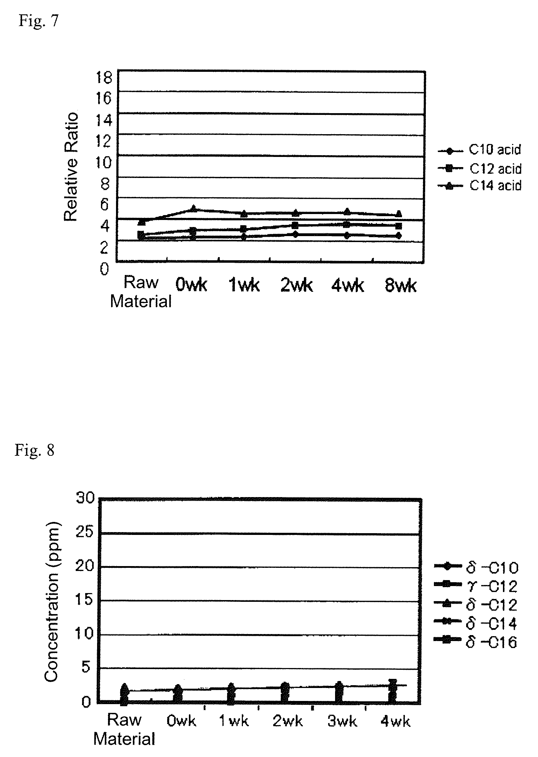

According to the analysis method of the present invention, it is possible to obtain a relative amount of free fatty acids in the tested butter oil. The data of changes in concentration of decanoic acid (C10 acid), dodecanoic acid(C12 acid), tetradecanoic acid (C14 acid) during storing Sample B at constantly 20°C are shown in Fig. 6. It was clearly demonstrated from Fig. 6 that tetradecanoic acid significantly increased. It was found that dodecanoic acid and decanoic acid also increased. On the other hand, in the data shown in Fig. 7, any increases in the fatty acids in Sample D were not found. Production of materials such as butanoic acid, hexanoic acid, and octanoic acid was not found in Sample B and Sample D.

### [Comparative Example 1]

The butter oil prepared by melting, centrifuging and rotary evaporator treatment (40°C) using (Sample D ; containing 757 ppm of moisture content) was stored at 5°C for 0, 1, 2, 3, or 4 weeks, which was subjected to the LDPE pouch extraction process and GC-MS analysis.

The results were shown in Fig. 8. According to data shown in Fig. 8, it was found that lactones were seldom produced at temperature of 5°C. It was confirmed that it was important to store the butter oil with being moisture adjusted at around in order to efficiently produce lactones. Also since the lactone production during the LDPE pouch extraction process was considered to be a problem in Comparative Example 2, pouch extraction was conducted after dehydration of the moisture content to 280 ppm or less.

### [Comparative Example 2]

To determine the effects of temperature on lactone production, Sample D was held at 40°C and 50°C for 1 hour, 2 hours, 4 hours, and 8 hours. These tested butter oil were directly subjected to the LDPE pouch extraction process and the GC-MS analysis. The results were shown in Fig. 9. When stored at 40°C, the lactone concentration tended to increase by 2 hours, while to mildly decline afterward. When stored at 50°C, as compared with the levels of lactones obtained at 40°C, the accumulated lactones was further lower, and the tendency became significant as the retention time prolongs. The above experimental data suggests that hydroxy fatty acid esters were affected by various intermolecular reactions at temperature of 40°C or higher where the butter oil is completely in the melting condition. That is to say, the data suggested that the ratio of the hydroxy fatty acid which was consumed in the pathway other than the lactone forming reaction increased under the melting condition.

### [Comparative Example 3]

The lactone potential of the tested butter oil (maximum lactone production amount) was determined by the following method. A combination of 20 g of the butter oil and 1.0 g of deionized water was collected in the pressure-resistant reservoir (inner diameter 15 mm x length 200 mm), was incubated at 160°C in a thermostated chamber for 100 minutes, to convert 4- and 5-hydroxy fatty acids into γ-lactone and δ-lactone, respectively. The butter oil decomposed material was subjected to the pouch extraction process to determine the lactone potential. The lactone potential (LP) of the butter oil used in this comparative example, the lactone concentration (L) obtained after storing Sample D at 20°C for 3 weeks, and the ratio of L/LP are shown in Table 1. Since the values of the ratio of L/LP for any of lactones are almost 1, the data suggested that almost all 4- and 5-hydroxy fatty acid in the butter oil were converted into the corresponding lactones by the present technique.
[Table 1]

**Table 1. The lactone potential (LP) the tested butter oil, the lactone concentration (L) of Sample D (20°C, 3 wk storage), and the ratio of L/LP**

| | lactone potential (LP) (ppm) | lactone concentration (L) (ppm) | Ratio of L/LP |
|---|---|---|---|
| | | Sample D (20°C, 3 wk) | |
| γ-dodecalactone | 3.1 | 3.0 | 0.97 |
| δ-decalactone | 9.3 | 9.1 | 0.98 |
| δ-dodecalactone | 20.6 | 20.9 | 1.01 |
| δ-tetradecalactone | 25.4 | 25.6 | 1.00 |
| δ-hexadecalactone | 17.0 | 16.5 | 0.97 |
| Total | 75.4 | 75.1 | 0.99 |

### [Example 3] Effect of addition of sucrose to the oil and fat composition on formation of lactones (1)

The butter oil was prepared by melting a commercially available unsalted butter at 40°C, and then centrifuging the melted butter. The moisture content of the butter oil was 1400 ppm, which was fall within the range of the moisture content of the butter oil which was generally distributed internationally. This butter oil sample was divided into 6 aliquots, to which 0.5% - 6.0% of sucrose were added to prepare Samples and nothing was added to prepare the control.

Table 2 shows lactone intensity and volatile fatty acid intensity yielded after storing the butter oil at 60°C for 1 or 2 hours with agitated as a sensory intensity. In the present example, it was found that amounts of lactones do not reduced after prolongation of heating at 60°C.

The sweet, milk-like aroma which is characteristic to lactones was strongly sensed in the sample added with 1.0% or more of sucrose. Little or no production of lactones was found in the sample added with no sucrose, or the sample added with 0.5% sucrose. On the other hand, irritating or rough feeling odor characteristic to volatile fatty acid was clearly sensed in the sample added with no sucrose. It was considered to be industrially useful that the butter oil added with sucrose generates less unusual odor after heating treatment.

Even after heating the butter oil added with sucrose at 60°C for 1 hour, excess sucrose existed in the particle state. Then, the particles of sucrose were subjected to centrifugation and precipitated, and the upper clear butter oil phase was subjected to Karl Fischer Titrator. As a result, values of the moisture content reduced depending on an amount of the added sucrose, which were all 900 ppm or more in any Samples.

When the butter oil containing 900 ppm or more of moisture content were heated at 60°C without adding sucrose, Fig. 9 clearly shows that, since not only the lactone production reaction but also lactone extinction reaction were both promoted, the accumulation of the lactones was not promoted.
[Table 2]

**Table 2: Intensity of lactone aroma (sensory intensity)**

| sucrose(%) | 0 | 0.5 | 1.0 | 2.0 | 4.0 | 6.0 |
|---|---|---|---|---|---|---|
| lactone intensity | 0 | 1 | 4 | 5 | 5 | 5 |
| fatty acid intensity | 4 | 1 | 0 | 0 | 0 | 0 |
| moisture content (ppm) | 1400 | 1200 | 1080 | 980 | 930 | 910 |

| | | | | | | |
|---|---|---|---|---|---|---|
| Values in Table 2: 0 None; 1 Low; 2 Not Low; 3 Considerably; 4 Strong; 5 Very Strong | | | | | | |

### [Example 3] Effect of addition of sucrose to the oil and fat composition on formation of lactones (2)

Sample G was prepared by added 4 weight % of sucrose to Sample B containing 1222 ppm of the moisture content which was prepared by centrifuging the oil and fat layer of the melted butter (3000 rpm, 3 minutes) and agitating it for 20 minutes to bind excessive moisture to sucrose molecules. The main part of the sucrose supplemented in this example existed at the bottom of the butter oil in the particle state. Sample G contains 1180 ppm of the moisture content, which was expected to be dispersed in the oil and fat being bound to sucrose molecules. Sample G was held at 60°C for 0.5, 1, 2, 3, or 4 hours, and the concentration of the lactones in the butter oil were traced.

The data of changes in concentration of δ-decalactone (δ-C10), γ-dodecalactone (y-C12), δ-dodecalactone (δ-C12), δ-tetradecalactone (δ-C14), and δ-hexadecalactone (δ-C16) during storing Sample G added with sucroseat 60°C for 0.5, 1, 2, 3, or 4 hours are shown in Fig. 10. Fig. 10 shows that the lactone concentration reached maximum amount after 3 hours incubation at 60°C, resulted in reaching total concentration 72 ppm.

Thus, it was found that when Sample G containing 1180 ppm of the moisture content was held at 60°C for 3 hours with sucrose, lactones were produced at almost the same amount of lactones which were obtained by storing Sample D (containing 757 ppm of moisture content) at 20°C for 3 weeks without adding sucrose. That is to say, the lactone concentration equivalent to the lactone potential was achieved by adding to the melted butter oil a component which has characteristics of forming hydrogen bonds with water molecules, such as monosaccharides (such as glucose or fluctose), disaccharides (such as sucrose), oligosaccharides, dextrins, starches and so on, as crystal particles or powder, and holding it at 40°C - 70°C for 1 hour - 10 hours. The similar effect was also found when the powdered sugar consisting of sucrose and cornstarch was used.

### [Comparative Example 4]

A significant increase in the lactone concentration was not found when Sample G which was prepared by centrifuging the oil and fat layer of the melted butter was held at 60°C for 0.5, 1, 2, 3, or 4 hours without adding sucrose.

## Claims

1. A method for producing an oil and fat composition enriched with lactones, which comprises steps a) and c) or b) and c):
a) keeping the oil and fat composition containing between 300 ppm and 1200 ppm of moisture content at the temperature where a solid fat exists in the oil and fat composition, or
b) adding to the oil and fat composition an additive agent which has an ability to form hydrogen bonds with water molecules,
keeping temperature of the oil and fat composition at the temperature between 40 and 60°C, and
c) non-enzymatically producing γ-lactone or δ-lactone from triglyceride containing 4- or 5-hydroxy fatty acid in the oil and fat composition.

2. The method for producing the oil and fat composition according to Claim 1, further comprising a step of placing the raw material oil and fat composition in the melted state under reduced pressure and/or a step of centrifuging the raw material oil and fat composition in the melted state to remove moisture to obtain the oil and fat composition containing between 300 ppm and 1200 ppm of moisture content.

3. The method for producing the oil and fat composition according to Claim 1, further comprising a step of adding moisture to the raw material oil and fat composition to obtain the oil and fat composition containing between 300 ppm and 1200 ppm of moisture content.

4. The method for producing the oil and fat composition according to any one of Claims 1-3, wherein the oil and fat composition is derived from butter.

5. The method for producing the oil and fat composition according to any one of Claims 1-4, wherein in step (a) the temperature is kept between 10°C and 35°C, or more preferably between 15°C and 30°C.

6. The method for producing the oil and fat composition according to any one of Claims 1-5, wherein in step (a) the oil and fat composition is held between 3 days and 2 months, or more preferably between 1 week and 4 weeks.

7. The method for producing the oil and fat composition according to Claim 1, wherein in step (b) the additive agent is sugar selected from at least one selected from the group consisting of monosaccharides, disaccharides, oligosaccharides, dextrins, and starches.

8. The method for producing the oil and fat composition according to Claim 7, wherein the sugar is sucrose.

9. The method for producing the oil and fat composition according to any one of Claims 1, 7 or 8, wherein in step (b) the oil and fat composition is held between 2 to 4 hours, or more preferably for 3 hours.

10. The method for producing the oil and fat composition according to any one of Claims 1-9, which is for the purpose of producing the oil and fat composition containing 50 ppm or more of lactones.

11. A method for producing a food product or a food product material, which comprises steps described in any one of Claims 1-10, wherein the oil and fat composition is enriched with lactones.

## Patentansprüche

1. Verfahren zur Erzeugung einer Öl- und Fettzusammensetzung, die mit Lactonen angereichert ist, umfassend die Schritte (a) und (c) oder (b) und (c):
(a) Halten der Öl- und Fettzusammensetzung, die zwischen 300 und 1200 ppm Feuchtigkeitsgehalt enthält, bei der Temperatur, bei der ein festes Fett in der Öl- und Fettzusammensetzung existiert, oder
(b) Zugabe eines Additives zu der Öl- und Fettzusammensetzung, das eine Fähigkeit zur Bildung von Wasserstoffbindungen mit Wassermolekülen hat,
Halten der Temperatur der Öl- und Fettzusammensetzung bei der Temperatur zwischen 40 und 60°C, und
(c) nicht-enzymatisches Erzeugen von γ-Lacton oder δ-Lacton von Triglycerid, enthaltend 4- oder 5-Hydroxyfettsäure, in der Öl- und Fettzusammensetzung.

2. Verfahren zur Erzeugung der Öl- und Fettzusammensetzung nach Anspruch 1, weiterhin umfassend den Schritt, dass die Öl- und Fettzusammensetzung als Ausgangsmaterial im geschmolzenen Zustand unter vermindertem Druck erhalten wird und/oder einen Schritt, dass die Öl- und Fettzusammensetzung als Ausgangsmaterial im geschmolzenen Zustand zentrifugiert wird, zur Entfernung von Feuchtigkeit, unter Erhalt der Öl- und Fettzusammensetzung, die einen Feuchtigkeitsgehalt zwischen 300 und 1200 ppm hat.

3. Verfahren zur Erzeugung der Öl- und Fettzusammensetzung nach Anspruch 1, weiterhin umfassend einen Schritt der Zugabe von Feuchtigkeit zu der Öl- und Fettzusammensetzung als Ausgangsmaterial, unter Erhalt der Öl- und Fettzusammensetzung, die einen Feuchtigkeitsgehalt zwischen 300 und 1200 ppm hat.

4. Verfahren zur Erzeugung der Öl- und Fettzusammensetzung nach einem der Ansprüche 1 bis 3, worin die Öl- und Fettzusammensetzung von Butter stammt.

5. Verfahren zur Erzeugung der Öl- und Fettzusammensetzung nach einem der Ansprüche 1 bis 4, worin im Schritt (a) die Temperatur zwischen 10 und 35°C und mehr bevorzugt zwischen 15 und 30°C gehalten wird.

6. Verfahren zur Erzeugung der Öl- und Fettzusammensetzung nach einem der Ansprüche 1 bis 5, worin im Schritt (a) die Öl- und Fettzusammensetzung zwischen 3 Tagen und 2 Monaten oder mehr bevorzugt zwischen 1 Woche und 4 Wochen gehalten wird.

7. Verfahren zur Erzeugung der Öl- und Fettzusammensetzung nach Anspruch 1, worin im Schritt (b) das Additivmittel Zucker ist, ausgewählt aus zumindest einem, ausgewählt aus der Gruppe bestehend aus Monosacchariden, Disacchariden, Oligosacchariden, Dextrinen und Stärken.

8. Verfahren zur Erzeugung der Öl- und Fettzusammensetzung nach Anspruch 7, worin der Zucker Sucrose ist.

9. Verfahren zur Erzeugung der Öl- und Fettzusammensetzung nach einem der Ansprüche 1, 7 oder 8, worin im Schritt (b) die Öl- und Fettzusammensetzung zwischen 2 bis 4 Stunden oder mehr bevorzugt 3 Stunden gehalten wird.

10. Verfahren zur Erzeugung der Öl- und Fettzusammensetzung nach einem der Ansprüche 1 bis 9, die zum Zweck der Erzeugung der Öl- und Fettzusammensetzung ist, enthaltend 50 ppm oder mehr Lactone.

11. Verfahren zur Erzeugung eines Nahrungsmittelproduktes oder eines Nahrungsproduktmaterials, umfassend die Schritte, beschrieben in einem der Ansprüche 1 bis 10, worin die Öl- und Fettzusammensetzung mit Lactonen angereichert ist.

## Revendications

1. Procédé de production d'une composition d'huile et de graisse enrichie en lactones, qui comprend les étapes a) et c) ou b) et c) de :
a) maintien de la composition d'huile et de graisse contenant entre 300 ppm et 1 200 ppm de teneur en humidité à la température où une graisse solide existe dans la composition d'huile et de graisse, ou
b) addition à la composition d'huile et de graisse d'un agent additif qui possède une capacité à former des liaisons hydrogène avec des molécules d'eau,
maintenir la température de la composition d'huile et de graisse à la température située entre 40 et 60 °C, et
c) production par voie non enzymatique de γ-lactone ou de δ-lactone à partir d'un triglycéride contenant de l'acide gras 4- ou 5-hydroxy dans la composition d'huile et de graisse.

2. Procédé de production de la composition d'huile et de graisse selon la revendication 1, comprenant en outre une étape consistant à placer la composition d'huile et de graisse matière première à l'état fondu sous pression réduite et/ou une étape consistant à centrifuger la composition d'huile et de graisse matière première à l'état fondu pour enlever l'humidité afin d'obtenir la composition d'huile et de graisse contenant entre 300 ppm et 1 200 ppm de teneur en humidité.

3. Procédé de production de la composition d'huile et de graisse selon la revendication 1, comprenant en outre une étape consistant à ajouter de l'humidité à la composition d'huile et de graisse matière première afin d'obtenir la composition d'huile et de graisse contenant entre 300 ppm et 1 200 ppm de teneur en humidité.

4. Procédé de production de la composition d'huile et de graisse selon l'une quelconque des revendications 1 à 3, dans lequel la composition d'huile et de graisse est dérivée du beurre.

5. Procédé de production de la composition d'huile et de graisse selon l'une quelconque des revendications 1 à 4, dans lequel, à l'étape (a), la température est maintenue entre 10 °C et 35 °C, ou plus préférentiellement entre 15 °C et 30 °C.

6. Procédé de production de la composition d'huile et de graisse selon l'une quelconque des revendications 1 à 5, dans lequel, à l'étape (a), la composition d'huile et de graisse est maintenue pendant une durée située entre 3 jours et 2 mois, ou plus préférentiellement entre 1 semaine et 4 semaines.

7. Procédé de production de la composition d'huile et de graisse selon la revendication 1, dans lequel, à l'étape (b), l'agent additif est un sucre choisi parmi au moins un élément sélectionné dans le groupe constitué par les monosaccharides, les disaccharides, les oligosaccharides, les dextrines et les amidons.

8. Procédé de production de la composition d'huile et de graisse selon la revendication 7, dans lequel le sucre est le saccharose.

9. Procédé de production de la composition d'huile et de graisse selon l'une quelconque des revendications 1, 7 ou 8, dans lequel, à l'étape (b), la composition d'huile et de graisse est maintenue pendant 2 à 4 heures, ou plus préférentiellement pendant 3 heures.

10. Procédé de production de la composition d'huile et de graisse selon l'une quelconque des revendications 1 à 9, qui est destiné aux fins de la production de la composition d'huile et de graisse contenant 50 ppm, ou plus, de lactones.

11. Procédé de production d'un produit alimentaire ou d'une matière de produit alimentaire, qui comprend les étapes définies selon l'une quelconque des revendications 1 à 10, dans lequel la composition d'huile et de graisse est enrichie en lactones.
